(19) Europäisches Patentamt

European Patent Office

Office européen des brevets

(11) **EP 0 686 139 B1**

(12) **EUROPÄISCHE PATENTSCHRIFT**

(45) Veröffentlichungstag und Bekanntmachung des Hinweises auf die Patenterteilung:
**12.05.1999 Patentblatt 1999/19**

(21) Anmeldenummer: **94908336.4**

(22) Anmeldetag: **18.02.1994**

(51) Int Cl.[6]: **C07C 53/126**, C07C 53/128, C07C 57/03, C07C 59/01, C07C 59/185, C07C 51/41

(86) Internationale Anmeldenummer:
**PCT/EP94/00458**

(87) Internationale Veröffentlichungsnummer:
**WO 94/19308 (01.09.1994 Gazette 1994/20)**

(54) **BASISCHE CALCIUM/ZINK-MISCHSEIFEN**

ALKALINE CALCIUM/ZINC MIXED SOAPS

SAVON MIXTES CALCIUM/ZINC BASIQUES

(84) Benannte Vertragsstaaten:
**AT DE FR GB IT NL**

(30) Priorität: **26.02.1993 DE 4305944**

(43) Veröffentlichungstag der Anmeldung:
**13.12.1995 Patentblatt 1995/50**

(73) Patentinhaber: **Henkel Kommanditgesellschaft auf Aktien**
**40191 Düsseldorf (DE)**

(72) Erfinder:
• **WORSCHECH, Kurt**
**D-27612 Loxstedt (DE)**
• **WEDL, Peter**
**D-27568 Bremerhaven (DE)**
• **JAECKEL, Manfred**
**D-27612 Loxstedt (DE)**
• **FLEISCHER, Erwin**
**D-27619 Schiffdorf (DE)**

(56) Entgegenhaltungen:
DE-A- 1 768 450          DE-C- 2 942 237

• **PATENT ABSTRACTS OF JAPAN vol. 7, no. 289 (C-202)(1434) 23 December 1983 & JP,A,58 167 538 (KOUSEI K.K.) 3 October 1983**

**Beschreibung**

Die Erfindung liegt auf dem Gebiet der thermoplastischen halogenhaltigen Kunststoffe und betrifft Stabilisatorzusammensetzungen für diese, die basische Calcium/Zink-Mischselfen gelöst oder suspendiert in Gleitmittel enthalten, sowie ein Verfahren zur Herstellung derartiger basischer Calcium/Zink-Mischseifen und ihre Verwendung als Stabilisatoren in thermoplastischen halogenhaltigen Kunststoffen.

Halogenhaltige Kunststoffe, insbesondere chlorhaltige wie Polyvinylchlorid (PVC), oder daraus hergestellte Formmassen neigen bekanntermaßen unter Hitzebedingungen oder beim Aussetzen an Ultraviolett-Licht zu einem Abbau bzw. zu Zersetzungserscheinungen, die sich beispielsweise in Form von Farbveränderungen äußern. Um den entgegenzuwirken, werden herkömmlicherweise Stabilisatormischungen zugesetzt, die Blei-, Zinn-, Barium- und/oder Cadmiumverbindungen enthalten. So sind beispielsweise aus der deutschen Offenlegungsschrift DE-A-34 44 259 basische Bleiseifensysteme des Typs 2Pb0 · Pb (Fettsäurerest)$_2$ bekannt. Aufgrund physiologischer Oberlegungen möchte man die bleihaltigen Stabilisatoren soweit wie möglich ersetzen. Dies gilt insbesondere für halogenhaltige thermoplastische Kunststoffe, die mit Lebensmitteln in Berührung kommen. Als Alternative zu bleihaltigen Stabilisatoren kommen beispielsweise Calcium- und Zinkverbindungen in Frage. Eine Übersicht über derartige Calcium/Zink-Stabilisatoren sind in Gächter/Müller, Kunststoff-Additive, 2. Ausgabe, Carl-Hanser-Verlag, München und Wien, Seiten 222 bis 224 (1983), zu finden. Bei den meisten Calcium/Zinkstabilisatoren handelt es sich um physikalische Mischungen von Calcium- und Zinkseifen von höheren Fettsäuren mit 8 bis 22 C-Atomen. Bei diesen Calcium-/Zinkseifen bewirkt der Fettsäurerest eine Gleitwirkung und die Metallkomponente die eigentliche Stabilisierung. Um eine hohe Stabilisierung zu erreichen, ist man auf der einen Seite daran interssiert, hohe Gehalte an diesen Seifen in die halogenhaltigen Kunststoffe einzutragen. Mit der Erhöhung des Eintrags an Metallseifen wird aber automatisch auch stets die Menge an eingetragenen Fettsäureresten erhöht, was aber zu ungewollten Überschmierungen führen kann. Desweiteren sind die Calcium- und Zinkseifen staubende Produkte, die unter anderem die mit der Handhabung betroffenen Menschen stören können.

Aufgrund der Überschmierungen wurde versucht, einen Teil der, insbesondere in der Schmelze stark schmierenden, Calciumseifen durch das nicht fettsäurehaltige Calciumoxid bzw. Calciumhydroxid zu ersetzen. Derartige Mischungen mit Zinkseifen zeigen aber leider keine geringere Schmierwirkung als Mischungen von Calcium- und Zinkseifen.

In der deutschen Offenlegungsschrift DE-A-3806192 wird vorgeschlagen, basische Metallseifen einer Zusammensetzung der Formel $(MO)_n \cdot M(RCOO)_2$, in der MO ein Oxid aus der von CaO, ZnO, MgO, BaO und PbO gebildeten Gruppe, M eines der vorgenannten Metalle und RCOO ein Fettsäurerest bedeuten, als Stabilisator/Gleitmittel-Zusammensetzung für die Kunststoffindustrie zu verwenden. Diese basischen Metallseifen werden erhalten, indem man pulverförmige Fettsäuren mit pulverförmigen Metalloxiden in Gegenwart von Wasser und/oder einer Säure umsetzt. Diese Festkörperreaktion führt jedoch zu Reaktionsmischungen, die noch große Mengen an unumgesetzten Edukten (z. B. CaO) neben geringen Mengen basischen Metallseifen enthalten. Damit sind auch diese Reaktionsmischungen nicht geeignet, Überschmierungserscheinungen im gewünschten Ausmaß zu vermeiden.

Die Aufgabe der vorliegenden Erfindung bestand darin, bleifreie Stabilisatoren zur Verfügung zu stellen, die bei einem hohen Gehalt an die eigentliche Stabilisierung bewirkenden Metallen einen möglichst niedrigen Gehalt an Fettsäureresten aufweisen. Die Stabilisatoren sollten zudem in hohen Ausbeuten zugänglich sein, damit keine bzw. in nur geringen Mengen Nebenprodukte zugegen sind, die eine Überschmierung bewirken könnten. Des weiteren sollten die Stabilisatoren möglichst wenig stauben und mit üblichen Additiven von halogenhaltigen Kunststoffen kompatibel sein.

Basische Calcium/Zink-Mischseifen einer Zusammensetzung der Formel (I), die durch Schmelzreaktion in Anwesenheit von für halogenhaltige Kunststoffe üblichen Gleitmittel, deren Schmelzen niedrigviskos sind, hergestellt worden sind, erfüllen diese Anforderungen.

Gegenstand der vorliegenden Erfindung ist die Verwendung basischer Calcium/Zink-Mischseifen der Formel (I)

$$(CaO)_n \cdot Zn(OOCR^1)_2 \qquad\qquad (I)$$

in der R[1] für einen oder mehrere Alkyl-, Alkenyl-, Hydroxyalkyl-, Hydroxyalkenylreste mit 7 bis 21 C-Atomen oder Ketoalkylreste mit 11 bis 21 C-Atomen steht und
n eine Zahl im Bereich von 0,1 bis 2,5 bedeutet,
wobei die basischen Calcium/Zink-Mischseifen gelöst oder suspendiert
in für halogenhaltige Kunststoffe üblichen Gleitmittel sind, hergestellt indem man Calciumoxid oder Calciumhydroxid zu einer Schmelze gibt, die Zinkseifen der Formel (III)

$$Zn \, (OOCR^1)_2 \qquad (III)$$

worin $R^1$ die oben angegebene Bedeutung hat - enthält und die für die thermoplastischen halogenhaltigen Kunststoffe übliche, in der Schmelze niedrigviskose Gleitmittel, wobei die Reaktionsmischung oberhalb ihres Schmelzpunktes in Gegenwart katalytischer Säuremengen bis zur Abreaktion des freien Calciumoxids oder Calciumhydroxids erhitzt wird und wobei man Calciumoxid oder Calciumhydroxid in einer Menge von 0,1 bis 2,5 Mol pro Mol Zinkseife (III) einsetzt, als Stabilisatoren für thermoplastische halogenhaltige Kunststoffe.

Die in der Formel (I) wiedergegebene Gruppe $R^1COO$- leitet sich ab von gesättigten und/oder ungesättigten Monocarbonsäuren mit 8 bis 22 C-Atomen oder von gesättigten oder ungesättigten Hydroxycarbonsäuren mit 8 bis 22 C-Atomen oder von Ketofettsäuren mit 12 bis 22 C-Atomen. Die Carbonsäuren und/oder Hydroxycarbonsäuren können natürlicher und/oder synthetischer Herkunft sein. Beispiele für geeignete Monocarbonsäuren sind Laurin-, Myristin-, Palmitin-, Stearin-, Arachin-, Behen-, Laurolein-, Myristolein-, Palmitolein-, Öl- und Erucasäure. Beispiele für geeignete Hydroxymonocarbonsäuren sind Ricinol- und 12-Hydroxystearinsäure. Besonders bevorzugt leitet sich die Gruppe $R^1COO$- von technischen Mischungen der genannten Fettsäuren ab, wie sie in Form der in der Fettchemie üblichen technischen Gemische nach Druckspaltung von Ölen und Fetten tierischer oder pflanzlicher Herkunft, wie Kokos-, Palmkern-, Sonnenblumen-, Raps-, Rübsen- und Korianderöl und Rindertalg, zugänglich sind. Die Gruppe $R^1COO$- kann aber auch einen verzweigten Fettsäurerest bedeuten, beispielsweise den Rest der 2-Ethylhexansäure, Isopalmitinsäure oder Isostearinsäure. Ebenso können die Carbonsäurereste $R^1COO$- sich von Ketofettsäuren mit 12 bis 22 C-Atomen ableiten. Typische und bevorzugte Vertreter dieser Ketofettsäuren sind die verschiedenen Isomeren der Ketostearinsäure, die in Acta Chemica Scandinavica 6, 1157 bis 1174 (1952) beschrieben sind. Von diesen isomeren Ketostearinsäuren sind die 4-, 9 (10)- und 12-Ketostearinsäuren besonders bevorzugt, da diese aus natürlichen Rohstoffen besonders leicht zugänglich sind.

Besonders bevorzugt steht in der Formel (I) die Gruppe $R^1COO$- für gesättigte Fettsäurereste und/oder Ketofettsäurereste der oben beschriebenen Fettsäuren bzw. Ketofettsäuren.

Gemäß einer vorteilhaften Ausführungsform der Erfindung bedeutet in der Formel (I) n eine ganze oder gebrochene Zahl im Bereich von 1 bis 1,5, das heißt, bevorzugte basische Calcium/Zink-Mischseifen weisen die Zusammensetzung $(CaO)_{1-1,5} \cdot Zn \, (OOCR^1)_2$ auf. Die genaue Strukturzusammensetzung der Calcium/Zink-Mischseifen der Formel (I) bzw. der bevorzugten Ausführungsformen ist nicht bekannt. Es ist aber sicher, daß es sich nicht um eine physikalische, sondern um eine chemische Mischung handelt, da freies Calciumhydroxid bzw. freies Calciumoxid nicht bzw. kaum mehr nachgewiesen werden konnte. Der Nachweis wurde durch Untersuchung der freien Mengen an Calciumoxid erbracht, die beobachtet wird, wenn man die in Gleitmittel gelösten oder suspendierten basischen Calcium/Zink-Mischseifen 3 Stunden im Wärmeschrank lagert. Ein Vergleich der erfindungsgemäßen basischen Calcium/Zink-Mischseifen mit den nach der DE-A-38 06 192 in einer Festkörperreaktion hergestellten zeigt, daß sich bei der Lagerung unterschiedlich viel freies Calciumoxid am Boden absetzt. Aufgrund der so durchgeführten Tests kann auf den Reinheitsgrad der basischen Calcium/Zink-Mischseifen geschlossen werden. Die erfindungsgemäßen Calcium/Zink-Mischseifen sind in über 85 gew.%iger Reinheit gelöst oder suspendiert in für halogenhaltige Kunststoffe üblichen Gleitmittel. Der zu 100 % fehlende Rest ist nichtumgesetztes Calciumoxid bzw. Calciumhydroxid - berechnet ohne Gleitmittel.

Die basischen Calcium/Zink-Mischseifen sind gelöst oder suspendiert in Gleitmittel, die an für sich für die thermoplastischen halogenhaltigen Kunststoffe üblich sind und die in der Schmelze niedrigviskos sind. Besonders bevorzugt werden Gleitmittel, die als Schmelze eine Viskosität nach Höppler bei 80 °C unter 150 mPa·s, aufweisen. Besonders bevorzugt sind die basischen Calcium/Zink-Mischseifen gelöst oder suspendiert in einem oder mehreren Gleitmittel ausgewählt aus der von

a) Fettalkoholen mit 8 bis 22 Kohlenstoffatomen,
b) Estern von monofunktionellen Alkanolen mit 1 bis 22 Kohlenstoffatomen mit Fettsäuren mit 8 bis 34 Kohlenstoffatomen,
c) Vollestern von Dicarbonsäuren mit 3 bis 6 Kohlenstoffatomen mit monofunktionellen Alkanolen mit 8 bis 22 Kohlenstoffatomen,
d) Partial- und Vollestern von Polyolen mit 2 bis 15 Kohlenstoffatomen und 2 bis 6 Hydroxylgruppen mit Fettsäuren mit 8 bis 34 Kohlenstoffatomen,
e) Fettketonen der Formel (II)

$$R^2\text{-CO-}R^3 \qquad (II)$$

in der $R^2$ und $R^3$, die gleich oder verschieden sein können und Alkylgruppen mit 5 bis 21 Kohlenstoffatomen bedeuten,

f) Diamiden von Fettsäuren mit 8 bis 22 Kohlenstoffatomen mit Alkylendiaminen mit 2 bis 6 Kohlenstoffatomen,

g) Kohlenwasserstoffwachse wie Polyethylenwachse und Paraffine

h) oxidierten Polyethylenwachsen mit einem Zahlenmittel der Molmassen im Bereich von 3000 bis 9000

gebildeten Gruppe.

Die aufgezählten Verbindungenden sind an sich bekannte Verbindungen für halogenhaltige Kunststoffe, die im Handel angeboten werden.

Besonders bevorzugt sind die basischen Calcium/ZInk-Mischseifen in den für thermoplastischen halogenhaltigen Kunststoffen üblichen Gleitmitteln der beschriebenen Art in Mengen von 10 bis 60 Gew.%, vorzugsweise von 20 bis 50 Gew.% - bezogen auf Mischung von basischen Calcium/Zink-Mischseifen und Gleitmittel - gelöst oder suspendiert enthalten. Der in diesem Zusammenhang gewählte Ausdruck, daß die Calcium/Zink-Mischseifen "gelöst" oder "suspendiert" enthalten sind, bedeutet, daß die basischen Calcium/Zink-Mischseifen in einer die üblichen Gleitmittel enthaltendenden Schmelze hergestellt worden sind.

Die basischen Calcium/Zink-Mischseifen können als alleinige Stabilisatorkomponente oder in Abmischung mit aus dem Stand der Technik bekannten Stabilisatoren verwendet werden. Derartige bekannte Stabilisatoren sind ggf. oberflächenmodifizierte Hydrotalcite, die unter dem Handelsnamen Alcamizer$^R$ von der Kyowa Chemical Int. vertrieben werden, synthetische kristalline Natriumsilikate, beispielsweise die in der deutschen Offenlegungsschrift DE-A-2412837 beschriebenen vom Typ NaA und/oder basische Calcium-Aluminium-Hydroxy-Phosphite gemäß der deutschen Patentschrift DE-C-3941902. Die Hydrotalcite, die von der Kyowa vertrieben werden, haben eine Oberfläche nach BET unter 30 $m^2$/g und sind gewöhnlich oberflächenmodifiziert mit Dispergiermitteln wie Natriumstearat. Man kann aber auch Hydrotalcite mit größeren Oberflächen oder mit anderen Oberflächenmodifizierungsmitteln verwenden, wie die in den deutschen Offenlegungsschriften DE-A-4117034 und DE-A-4117035 beschriebenen Hydrotalcite. Des weiteren können als Stabilisatoren basische Magnesium/Zink-Mischseifen einer Zusammensetzung der Formel $(MgO)_x Zn(OOCR)_2$, wobei x eine Zahl von 0,1 bis 2,5 und R ein Alkylrest bedeuten kann, verwendet werden. Derartige basische Magnesium/Zink-Mischseifen werden gemäß einer älteren Anmeldung der Anmelderin in einer Schmelzreaktion aus Zinkseifen der Formel $Zn(OOCR)_2$ und 0,1 bis 2,5 Mol Magnesiumoxid oder - hydroxid pro mol Zinkseife hergestellt, vorzugsweise in Anwesenheit katalytischer Mengen an Säuren und insbesondere bei Temperaturen über der Schmelztemperatur und unter Zersetzungstemperatur der Zinkseife. Besonders bevorzugt werden die erfindungsgemäßen basischen Calcium/Zink-Mischseifen zusammen mit den Hydrotalciten und/oder basischen Magnesium/Zink-Mischseifen verwendet.

Calcium/Zink-Mischseifen der Formel (I) lassen sich herstellen, in dem man Calciumoxid oder Calciumhydroxid zu einer Schmelze gibt, die Zinkseifen der Formel (III) $Zn(OOCR^1)_2$ mit der bereits gegebenen Bedeutung für $R^1$ enthält und die für die thermoplastischen halogenhaltigen Kunststoffe üblichen, in der Schmelze niedrigviskosen Gleitmittel, wobei die Reaktionsmischung oberhalb ihres Schmelzpunktes in Gegenwart katalytischer Säuremengen bis zur Abreaktion des freien Calciumoxids oder Calciumhydroxids erhitzt wird. Calciumoxid oder Calciumhydroxid wird in Mengen von 0,1 bis 2,5 Mol pro Mol Zinkseife der Formel (III) der Schmelze zugegeben. In der Regel werden gleichzeitig mit der Zugabe des Calciumoxids oder Calciumhydroxids die katalytischen Mengen an Säuren zugegeben. Die erhaltene Reaktionsmischung wird solange in der Schmelze gehalten, bis das Calciumoxid oder Calciumhydroxid nahezu vollständig abreagiert hat. Das Ende der Reaktion ist dann erreicht, wenn eine klare oder trübe Schmelze auftritt, die keine mit den Augen noch zu erkennenden Feststoffteilchen mehr enthält. Der Umsetzungsgrad kann wie oben beschrieben durch Bestimmung des nicht umgesetzten Calciumoxids bzw. Calciumhydroxids bei Lagerung bestimmt werden. Besonders bevorzugt werden 1 bis 1,5 Mol Calciumoxid oder Calciumhydroxid pro Mol Zinkseife zugegeben. Des weiteren ist es bevorzugt, in Gegenwart von Säuren in Mengen von 0,001 bis 0,1 Gew.-% - bezogen auf Reaktionsmischung - zu arbeiten. Typische Säuren sind kurzkettige Monocarbonsäuren mit 1 bis 3 Kohlenstoffatomen, wie Essigsäure, sowie Mineralsäuren, deren Anion mit den zu stabilisierenden Kunststoffmassen verträglich sind, wie Phosphorsäure. Die Temperatur, bei der die Umsetzung zwischen Calciumhydroxid oder Calciumoxid mit den Zinkseifen in Anwesenheit der geschilderten üblichen Mitteln erfolgt, liegt über den Schmelztemperaturen und unter den Zersetzungstemperaturen der Zinkseifen. In der Regel liegen die Umsetzungstemperaturen im Bereich von 100 bis 180 °C, je nach dem Schmelzpunkt und Zersetzungspunkt der gewählten Zinkseife. Für Umsetzungen von Zinkstearat liegen die Temperaturen etwa im Bereich von 150°C. Die Umsetzungen des Calciumoxids bzw. Calciumhydroxids mit den Zinkseifen wird in Anwesenheit der bereits genannten Gleitmittel durchgeführt, um die Viskosität der Schmelze zu erniedrigen. So würde ohne Anwesenheit dieser Verbindungen eine bei der Schmelztemperatur der Zinkseifen hochviskose Reaktionsmischung entstehen, die so gut wie nicht mehr zu rühren ist, so daß die Umsetzung nicht bzw. nur teilweise zu Ende zu führend ist. Die Mengenverhältnisse an in der Schmelze enthaltenen Gleitmittel wird so gewählt, daß 40 bis 90 Gew.%, vorzugsweise 50 bis 80 Gew.% der erhaltenen Mischung Gleitmittel sind, 10 bis 60 Gew.%, vorzugsweise 20 bis 50 Gew.% der Mischung sind die basischen Calcium/Zink-Mischseifen.

Die in dem Verfahren eingesetzten Zinkseifen der Formel (III) können entweder im Handel erstanden werden, oder man kann sie auch in situ selbst erzeugen, indem man Carbonsäuren, Hydroxycarbonsäuren oder Ketofettsäuren der Formel $R^1COOH$ mit Zinksalzen, vorzugsweise mit Zinkoxid, in einem molaren Verhältnis von etwa 2:1 umsetzt. Diese Umsetzung wird vorzugsweise so durchgeführt, daß zunächst die Carbonsäuren $R^1COOH$ geschmolzen werden und in diese Schmelze die Zinksalze, insbesondere das Zinkoxid, zugegeben werden. Die Umsetzung wird solange geführt, bis sich die Fettsäuren praktisch vollständig umgesetzt haben. Die praktisch vollständige Umsetzung kann man erkennen durch Ermittlung der Säurezahl gemäß DIN 53402. Die Umsetzung kann gleichermaßen in Gegenwart von katalytischen Mengen an Säuren beschleunigt werden, wobei die Säuremenge im Bereich von 0,001 bis 0,1 Gew.-% - bezogen auf Reaktionsmischung - betragen kann und ebenfalls kurzkettige Monocarbonsäuren sowie Mineralsäuren eingesetzt werden.

Die bevorzugte Ausführungsform des geschilderten Verfahrens ist es, die Carbonsäuren $R^1COOH$ zusammen mit den Gleitmitteln vorzulegen und zu schmelzen. Zu dieser Schmelze werden die Zinksalze in solchen stöchiometrischen Mengen zugegeben, daß sich zunächst Zinkseifen der Formel (III) bilden. Bevorzugt wird in Anwesenheit von katalytischen Säuremengen gearbeitet. Zu der so erhaltenen Schmelze aus Zinkseifen und Gleitmitteln wird anschließend das Calciumoxid bzw. Calciumhydroxid in entsprechenden molaren Mengen gegeben. Auch bei diesem zweiten Reaktionsschritt wird vorzugsweise in Anwesenheit von katalytischen Säuremengen gearbeitet. Sofern Calciumhydroxid zugegeben wird, wird entstehendes Reaktionswasser aus der Reaktionsmischung laufend entfernt. Nach Beendigung der Reaktion fallen die basischen Calcium/Zink-Mischseifen in den üblichen Gleitmitteln vorzugsweise in den Gleitmitteln gemäß a) bis h), gelöst oder suspendiert an. Das erhaltene Reaktionsprodukt ist ein nicht-staubendes Festprodukt, das wenn gewünscht, weiter konfektioniert werden kann, beispielsweise durch Sprühung.

Sofern die erfindungsgemäß einzusetzenden basischen Calcium-Mischseifen mit weiteren aus dem Stand der Technik bekannten Stabilisatoren wie die Hydrotalcite oder basischen Magnesium/Zink-Mischseifen verwendet werden, so werden diese erst zu der fertigen Mischung von basischen Calcium/Zink-Mischseifen und üblichen Gleitmitteln, vorzugsweise gemäß a) bis h), zugemischt.

Sofern noch weitere aus dem Stand der Technik bekannte Stabilisatoren zusammen mit den erfindungsgemäß einzusetzenden basischen Calcium/Zink-Mischseifen verwendet werden, werden diese in üblichen Mengen eingesetzt, so können beispielsweise die Hydrotalcite in Mengen von 0,1 bis 0,5 Gewichtsteile und die basischen Calcium/Zink-Mischseifen in Mengen von 0,05 bis 5 Gewichtsteilen auf 100 Gewichtsteile halogenhaltige Kunststoffe verwendet werden. Falls gewünscht, können zusätzlich weitere aus dem Stand der Technik bekannte Gleitmittel, Trennmittel, Weichmacher und/oder Co-Stabilisatoren wie die bereits unter a) bis h) genannten Gleitmittel oder Ester von epoxidierten Fettsäuren mit monofunktionellen Alkanolen oder mit Polyolen, β-Diketonen wie Benzoylstearoylmethan, hydroxylgruppenhaltige Isocyanurate und/oder sekundäre und/oder tertiäre Ester der phosphorigen Säure wie Dialkylarylphosphite. Diese zusätzlichen Additiven können in üblichen Mengen mit zu den thermoplastischen halogenhaltigen Kunststoffen gegeben werden.

Als halogenhaltige Kunststoffe kommen in erster Linie chlorhaltige Homo- und Copolymere von Vinylchlorid in Betracht. Als Copolymere werden bevorzugt Polymerisate von mindestens 50 Gew.% Vinylchlorid und weiteren polymerisierbaren Monomeren wie den Vinylester, Methacrylsäureester, Fumarsäureester, Butadien und Vinylidenchlorid. Besonders bevorzugt werden jedoch Polyvinylchloridhomopolymerisate mit K-Werten von 60 bis 70, die durch Emulsions-, Suspensions- und Massepolymerisation hergestellt werden können. Selbstverständlich kann das PVC auch mit Schlagzähigkeitsverbesserern und Flowmodifiern modifiziert sein.

Bei Verwendung der erfindungsgemäß einzusetzenden Stabilisatoren zeigen insbesondere PVC-Massen trotz des für die Stabilisierung erforderlichen Metallgehalts an Calcium und Zink keine Überschmierungen. Dies ist insbesondere im Extruder zu sehen, wo der Druckaufbau geringer abfällt als für Mischungen von Calciumoxid und Zinkseifen oder von Calciumseifen und Zinkseifen. Daher kann man sehr viel mehr an den erfindungsgemäßen Stabilisatoren in dem Kunststoff verwenden, ohne daß man Überschmierungen befürchten müßte. Letztlich sind die erfindungsgemäßen basischen Calcium/Zink-Mischseifen nicht-staubende Feststoffe, die gut konfektioniert werden können und leicht handhabbar sind.

**Beispiele**

**A) Herstellung der basischen Calcium/Zink-Seifen %-Angaben sind Gewichtsprozente**

**Beispiel 1: CaO-Zn-2-ethylhexanoat (40%) mit Glycerindistearat (60%)**

In einen 4-Halsrundkolben versehen mit Rührer, Thermometer und absteigendem Kühler wurden 56,7 g (0,39 Mol) 2-Ethylhexansäure, und 120 g Glycerindistearat gegeben. In das geschmolzene Gemisch wurde portionsweise 15,9 g (0,195 Mol) Zinkoxid gegeben und anschließend Vakuum angelegt. Das Vakuum wurde kontinuierlich verstärkt bis nach 1 Stunde etwa 30 hPa erreicht waren. Nach insgesamt 1 1/2 Stunden war eine Säurezahl gemäß DIN 53402 von unter 4 erreicht. Nach Belüftung wurde im zweiten Reaktionsschritt 14,4 g (0,195 Mol) Calciumhydroxid und 0,05 g 99 %ige Essigsäure zugesetzt. Nach 2 1/2 Stunden bei einem Vakuum von etwa 30 hPa war die Reaktion beendet. Man erhielt eine gelbstichige, sprödwachsige Masse mit einem Schmelzpunkt von 75 °C.

**Beispiel 2: CaO-Zinkoleat (40%) mit Glycerindioleat (60%)**

Analog Beispiel 1 wurden 65,9 g (0,24 Mol) technische Ölsäure, 120 g Glycerindioleat und 0,05 g 99 %ige Essigsäure vorgelegt. In das geschmolzene Gemisch wurde portionsweise 9,6 g (0,12 Mol) Zinkoxid eingetragen und Vakuum angelegt. Im zweiten Reaktionsschritt wurden 8,7 g (0,12 Mol) Calciumhydroxid und 0,05 g Essigsäure zugegeben und wie in Beispiel 1 weiterverfahren. Man erhielt eine gelblich-braunstichige, vaselinartige Masse mit einem Schmelzpunkt von 69 °C.

**Beispiel 3: CaO-Zn-12-Hydroxystearat (40%) mit gehärtetem Ricinusöl (60%)**

Analog Beispiel 1 wurden 67,2 g (0,22 Mol) 12-Hydroxystearinsäure, 120 g gehärtetes Ricinusöl und 0,05 99 %ige Essigsäure vorgelegt. Zu der geschmolzenen Mischung wurden 8,7 g (0,11 Mol) Zinkoxid portionsweise zugegeben und Vakuu angelegt. Im zweiten Verfahrensschritt wurden 8,0 g (0,11 Mol) Calciumhydroxid und 0,05 g Essigsäure zugegeben und analog Beispiel 1 weiterverfahren. Man erhielt eine bräunliche, wachsige Masse mit einem Schmelzpunkt von 132 °C.

**Beispiel 4: CaO-Znstearat (40%) mit Glycerintristearat (60%)**

Analog Beispiel 1 wurden 6,5 g (0,24 Mol) technische Stearinsäure, 120 g Glycerintristearat und 0,05 g 99 %ige Essigsäure vorgelegt. Zu der geschmolzenen Mischung wurden 9,9 g (0,12 Mol) Zinkoxid portionsweise zugegeben und Vakuum angelegt. Analog Beispiel 1 wurden im zweiten Verfahrensschritt 9,0 g (0,12 Mol) Calciumhydroxid so wie 0,05 g Essigsäure zugegeben und wie in Beispiel 1 weiterverfahren. Man erhielt eine braunstichige, hartwachsige Masse mit einem Schmelzpunkt von 106 °C.

**Beispiel 5: CaO-Znstearat (40%) mit Talgfettalkohol (60%)**

Analog Beispiel 1 wurden 65,5 g (0,24 Mol) technische Stearinsäure, 120 g Talgfettalkohol und 0,05 g 99 %ige Essigsäure vorgelegt. Zu der geschmolzenen Mischung gab man 9,9 g (0,12 Mol) Zinkoxid und legte Vakuum an. Im zweiten Reaktionsschritt wurden 9,0 g (0,12 Mol) Calciumhydroxid sowie 0,05 g Essigsäure zugegeben und wie in Beispiel 1 beschrieben umgesetzt. Man erhielt eine gelbstichige, wachsartige Masse mit einem Schmelzpunkt von 108 °C.

**Beispiel 6: CaO-Znstearat (40%) mit Talgfettalkoholstearat**

Analog Beispiel 1 wurden 65,5 g (0,24 Mol) technische Stearinsäure, 120 g Talgfettalkoholstearat und 0,05 g 99 %ige Essigsäure vorgelegt. Zu der geschmolzenen Mischung wurden 9,9 g (0,12 Mol) Zinkoxid portionsweise zugegeben und Vakuum angelegt. Im zweiten Reaktionsschritt wurden 9,0 g (0,12 Mol) Calciumhydroxid und 0,05 g Essigsäure zugegeben und wie in Beispiel 1 weiterverfahren. Man erhielt eine gelbstichige, hartwachsige Masse mit einem Schmelzpunkt von 115 °C.

**Beispiel 7: 1.5 CaO-Znstearat (20%) mit Pentaerythritdistearat (60%)**

Analog Beispiel 1 wurden 31,4 g (0,116 Mol) technische Stearinsäure, 80 g Penta R-di-stearat und 0,05 Essigsäure vorgelegt. In die geschmolzene Mischung wurden 4,7 g (0,058 Mol) Zinkoxid portionsweise zugegeben und Vakuum angelegt. Im zweiten Reaktionsschritt wurden weitere 80 g Penta R-di-stearat in die Reaktionsmischung gegeben und nach dem dieses aufgeschmolzen war zusätzlich 6,5 g (0,088 Mol) Calciumhydroxid und 0,05 g Essigsäure. Die Umsetzung wurde wie in Beispiel 1 weitergeführt. Man erhielt eine gelbstichige wachsige Masse mit einem Schmelzpunkt von 110 °C.

**Beispiel 8: CaO-Znstearat (40%) mit Glycerindistearat (60%)**

Analog Beispiel 1 wurden 65,5 g (0,24 Mol) technische Stearinsäure, 120 g Glycerindistearat und 0,05 g 99 %ige Essigsäure vorgelegt. In das geschmolzene Gemisch wurde portionsweise 9,8 g (0,12 Mol) Zinkoxid eingetragen und Vakuum angelegt. Im zweiten Reaktionsschritt wurden 9,1 g (0,12 Mol) Calciumhydroxid und 0,05 g Essigsäure zugegeben und weiterverfahren wie in Beispiel 1. Man erhielt eine schwach gelbliche, wachsige Masse mit einem Schmelzpunkt von 95 °C.

**Beispiel 9: CaO-Znstearat (30%) mit Paraffin FP 90-100 (70%)**

Analog Beispiel 1 wurden 65,5 g (0,24 Mol) technische Stearinsäure, 186,7 g Paraffin und 0,05 g Essigsäure vorgelegt. Zu der geschmolzenen Mischung wurden 9,9 g (0,12 Mol) Zinkoxid portionsweise zugegeben und Vakuum angelegt. In der zweiten Reaktionsstufe wurden 9,0 g (0,12 Mol) Calciumhydroxid und 0,05 g Essigsäure zugegeben und analog Beispiel 1 weiterbehandelt. Man erhielt eine gelbstichige, wachsige Masse mit einem Schmelzpunkt von 127 °C.

**Beispiel 10: Ca0-Zn-12-Ketostearat (50 %) mit Glycerindistearat (50%)**

Analog Beispiel 1 wurden 83,4 g (0,28 Mol) 12-Ketostearinsäure, 100 g Glycerindistearat und 0,05 g 99 %ige Essigsäure vorgelegt. Zur Schmelze wurden 11,3 g (0,14 Mol) Zinkoxid portionsweise eingetragen und Vakuum angelegt. Im zweiten Reaktionsschritt wurden 10,3 g (0,14 Mol) Calciumhydroxid sowie 0,05 g Essigsäure zugegeben und die Reaktion analog Beispiel 1 weitergeführt. Man erhielt eine gelbstichige, schwachhartwachsige Masse mit einem Schmelzpunkt von 115 °C.

**Beispiel 11: 2 CaO-Znstearat (40%) mit Glycerindistearat (60%)**

Analog Beispiel 1 wurden 60,4 g (0,22 Mol) technische Stearinsäure, 120 g Glycerindistearat und 0,05 g 99 %ige Essigsäure vorgelegt. In das geschmolzene Gemisch wurden bei 140 bis 150 °C portionsweise 9,1 g (0,11 Mol) Zinkoxid eingetragen und Vakuum angelegt. Im zweiten Reaktionsschritt wurden 16,6 g (0,22 Mol) Calciumhydroxid sowie 0,05 g 99 %ige Essigsäure zugegeben und die Reaktion analog Beispiel 1 weitergeführt.
Man erhielt eine schwach-gelbliche wachsige Masse mit einem Schmelzpunkt von 95 °C.

**B) Vergleich der freien Menge an Calciumoxid**

B1) 150 g der erfindungsgemäßen basischen Calcium/Zink-Mischseife nach Beispiel A8), entsprechend 40 % CaO · Zinkstearat in 60 % Glycerindistearat

B2) 60 g der basischen Calcium/Zink-Mischseife gemäß Beispiel 1 der DE-A-38 06 192 wurden in 90 g Glycerindistearat homogenisiert bis eine feinteilige homogene Feststoffmischung vorlag

B1) und B2) wurden aufgeschmolzen und in ein Becherglas überführt. Das Becherglas wurde 3 Stunden in einem Wärmeschrank bei 140 °C gelagert. Nach Abkühlung der geschmolzenen Masse wurde das Becherglas entfernt. Von der erstarrten Masse wurde oben, in der Mitte und vom Bodensatz Produkt abgeraspelt. Das geraspelte Produkt wurde auf seinen Aschegehalt nach DIN EN7, d. h. Calcium- und Zinkoxid untersucht und zu der theoretischen Menge an Zinkoxid und Calciumoxid (8,4 %) in Beziehung gesetzt.
Bei B1) wurde im geschmolzenen Zustand keine Phasentrennung bemerkt.
Die Bestimmung des Aschegehalts ergab folgende Mengen an Calcium- und Zinkoxid:
oben: 8,2 %        Mitte: 8,2 %        unten: 8,7 %. D. h. in Bezug zum theorethischen Gehalt an Calcium- und Zinkoxid von 8,4 % hat sich aus der Schmelze 8,4 - 8,2 % = 0,2 % abgesetzt. Unter der Annahme das der gesamte Bodensatz Calciumoxid ist und von den 8,4 % Calcium- und Zinkoxiden 3,4 % Calciumoxid ist, ergibt sich über die Beziehung $\frac{0,2 \cdot 100}{3,4} = 5,8$ ein Gehalt von 5,8 % freiem Calciumoxid, was sich als Bodensatz ansammeln könnte bzw. ein Umsetzungsgrad von 100 % - 5,8 % = 94,2 % an basischen Calcium/Zink-Mischseifen.
Bei B2) konnte man schon nach Lagerung der Schmelze ansehen, daß sich unter Aufklaren der überstehenden Schmelze ein dicker Bodensatz abgesetzt hat. Die Bestimmung des Aschegehalts ergab folgende Mengen an Calcium- und Zinkoxid:
oben: 5,6 %; Mitte: 5,6 %; Bodensatz: 21,7 %, d. h. in Bezug zum theoretischen Gehalt von 8,4 % an Calcium- und Zinkoxid hat sich aus der Schmelze 8,4 - 5,6 = 2,8 % abgesetzt. Da dies nicht alles Calciumoxid sein konnte, wurde der Bodensatz titriert mit Titriplex R 3, um den Gehalt an Calcium zu bestimmen. Umgerechnet auf Calciumoxid wurden 1,17 % gefunden, d. h. $\frac{1,17 \% \cdot 100 \%}{3,4} = 34,4$ % der theoretisch vorhandenen Menge an Calciumoxid sind als freies Calciumoxid im Produkt B2) enthalten.

## C) Anwendungstests

100 Gewichtsteile Suspension-PVC mit einem K-Wert von 68 (Corvic S 68/173$^R$) wurden vermischt mit den in Tabelle 1 aufgeführten Verbindungen in den dort aufgeführten Mengen (Mengenangabe in Gewichtsteile; phr). In den erfindungsgemäßen Beispielen C1) und C2) wurde CaO-Zinkstearat (40 %) in Glycerindistearat (60 %) gemäß Beispiel A8) getestet. Als Vergleich wurde eine Mischung von Calciumstearat und Zinkstearat sowie Calciumhydroxid und Zinkstearat gewählt. Die Menge an Vergleichsmischung wurde so gewählt, daß der Ca- und Zinkgehalt dem des Beispiels A8) entspricht.

Tabelle 1:

| PVC-Zusammensetzung | | | | | | |
|---|---|---|---|---|---|---|
| Produkte | C1 | C2 | Vgl. 1 | Vgl. 2 | Vgl. 3 | Vgl. 4 |
| PVC | 100 | 100 | 100 | 100 | 100 | 100 |
| Schlagzähigkeitsverbesserer und Flowmodifier auf Acrylatbasis (Paraloid$^R$) | 9 | 9 | 9 | 9 | 9 | 9 |
| Titandioxid (Kronos 2220$^R$) | 4 | 4 | 4 | 4 | 4 | 4 |
| Kreide (Omyalite 95 T$^R$) | 4 | 4 | 4 | 4 | 4 | 4 |
| Hydrotalcit (Alcamizer 4$^R$) | 0,6 | 0,6 | 0,6 | 0,6 | 0,6 | 0,6 |
| Alkylarylphosphit | 0,3 | 0,3 | 0,3 | 0,3 | 0,3 | 0,3 |
| Trishydroxyethylcyanurat | 0,3 | 0,3 | 0,3 | 0,3 | 0,3 | 0,3 |
| Benzoylstearylmethan | 0,1 | 0,1 | 0,1 | 0,1 | 0,1 | 0,1 |
| Glycerindistearat | 0,1 | 0,55 | 1 | 1 | 1 | 1 |
| Cetylstearylpalmitatstearat | 0,5 | 0,5 | 0,5 | 0,5 | 0,5 | 0,5 |
| Beispiel A8) | 1,5 | 0,75 | - | - | - | - |
| Zinkstearat · 2 MgO | - | 0,25 | - | - | - | - |
| Calciumstearat | - | - | 0,53 | - | 0,26 | - |
| Zinkstearat | - | - | 0,55 | 0,55 | 0,5 | 0,55 |
| Calciumhydroxid | - | - | - | 0,05 | - | - |
| Calciumoxid | - | - | - | - | - | 0,05 |
| Magnesiumstearat | - | - | - | - | 0,4 | - |

Die in Tabelle I wiedergegebene Zusammensetzung wurde zu einer Formmasse verarbeitet. Bei der Verarbeitung der Formmasse wurde ein handelsüblicher Doppelschneckenextruder (Reifenhäuser Doppelschneckenextruder BT 55/16) verwendet. Bei der Herstellung von Vierkantrohren (63x3 mn) wurden folgende Parameter gewählt:

Zylindertemperatur: 170 / 170 / 170 / 150 / 150 °C
Kopftemperatur: 155 / 170 / 170 °C
Schnecke: K 6/2
Schneckendrehzahl: 25 Upm.

Während der Verarbeitung wurde der Massedruck 2 (in bar) bestimmt, der sich vor dem formgebenden Werkzeug aufbaut. Des weiteren wurde die Maschinenauslastung (in %) abgelesen. Die Ergebnisse sind in Tabelle 2 zusammengestellt.

Tabelle 2:

| Parameter | C1) | C2) | Vgl. 1 | Vgl. 2 | Vgl. 3 |
|---|---|---|---|---|---|
| Massedruck 2 | 319 | 301 | 270 | 270 | 249 |
| Auslastung | 51 | 46 | 42 | 36 | 39 |

Tabelle 2 zeigt, daß der Massedruck vor dem formgebenden Werkzeug bei den erfindungsgemäßen Beispielen deutlich höher ist als bei den Vergleichsbeispielen. Dies sagt bei guter Maschinenauslastung aus, daß eine gute Gleitwirkung vorhanden ist, aber keine Überschmierung auftritt (kleiner Massedruck 2).

Des weiteren wurde die dynamische Stabilität an 0,5 mm dicken Walzfellen (1 x 1 cm große) geprüft. Dazu wurden die 1 x 1 cm großen Prüfstücke in einen Wärmeschrank mit rotierenden Horden bei 180 °C gegeben. Nach jeweils 15 Minuten wurden Proben entnommen. Bei Auftreten einer Dunkelbraunfärbung ist Stabilitätsende erreicht. Getestet wurden die in Tabelle 1 angegebenen Zusammensetzungen C1) und Vgl. 4).

Tabelle 3:

| Beispiel | Stabilitätsabbruch in Minuten |
|----------|-------------------------------|
| C 1      | 135                           |
| Vgl. 2   | 120                           |

Tabelle 3 zeigt, daß die dynamischen Stabilitäten der Beispiele mit den erfindungsgemäßen Stabilisatoren besser sind als Mischungen von Zinkseifen und Calciumoxid (Vgl. 4).

**Patentansprüche**

1.  Verwendung basischer Calcium/Zink-Mischseifen der Formel (I)

$$(CaO)_n \cdot Zn(OOCR^1)_2 \qquad (I)$$

in der $R^1$ für einen oder mehrere Alkyl-, Alkenyl-, Hydroxyalkyl-, Hydroxyalkenylreste mit 7 bis 21 C-Atomen oder Ketoalkylreste mit 11 bis 21 C-Atomen steht und
n eine Zahl im Bereich von 0,1 bis 2,5 bedeutet,
wobei die basischen Calcium/Zink-Mischseifen gelöst oder suspendiert
in für halogenhaltige Kunststoffe üblichen Gleitmittel sind, hergestellt indem man Calciumoxid oder Calciumhydroxid zu einer Schmelze gibt, die Zinkseifen der Formel (III)

$$Zn\,(OOCR^1)_2 \qquad (III)$$

worin $R^1$ die oben angegebene Bedeutung hat - enthält und die für die thermoplastischen halogenhaltigen Kunststoffe übliche, in der Schmelze niedrigviskose Gleitmittel, wobei die Reaktionsmischung oberhalb ihres Schmelzpunktes in Gegenwart katalytischer Säuremengen bis zur Abreaktion des freien Calciumoxids oder Calciumhydroxids erhitzt wird und wobei man Calciumoxid oder Calciumhydroxid in einer Menge von 0,1 bis 2,5 Mol pro Mol Zinkseife (III) einsetzt, als Stabilisatoren für thermoplastische halogenhaltige Kunststoffe.

2.  Verwendung nach Anspruch 1, dadurch gekennzeichnet, daß man solche basischen Calcium/Zink-Mischseifen der Formel (I) einsetzt, bei denen n für eine Zahl im Bereich von 1 bis 1,5 steht.

3.  Verwendung nach einem der Ansprüche 1 oder 2, dadurch gekennzeichnet, daß die basischen Calcium/Zinkseifen (I) in einer oder mehreren Verbindungen ausgewählt aus der von

a) Fettalkoholen mit 8 bis 22 Kohlenstoffatomen,
b) Estern von monofunktionellen Alkanolen mit 1 bis 22 Kohlenstoffatomen mit Fettsäuren mit 8 bis 34 Kohlenstoffatomen,
c) Vollestern von Dicarbonsäuren mit 3 bis 6 Kohlenstoffatomen mit monofunktionellen Alkanolen mit 8 bis 22 Kohlenstoffatomen,
d) Partial- und Vollestern von Polyolen mit 2 bis 15 Kohlenstoffatomen und 2 bis 6 Hydroxylgruppen mit Fettsäuren mit 8 bis 34 Kohlenstoffatomen,
e) Fettketonen der Formel (II)

$$R^2\text{-}CO\text{-}R^3 \qquad (II)$$

in der $R^2$ und $R^3$, die gleich oder verschieden sein können und Alkylgruppen mit 5 bis 21 Kohlenstoffatomen bedeuten,
f) Diamiden von Fettsäuren mit 8 bis 22 Kohlenstoffatomen mit Alkylendiaminen mit 2 bis 6 Kohlenstoffatomen,

g) Kohlenwasserstoffwachse wie Polyethylenwachse und Paraffinen

h) oxidierten Polyethylenwachsen mit einem Zahlenmittel der Molmassen im Bereich von 3000 bis 9000

gebildeten Gruppe gelöst oder suspendiert vorliegen.

**4.** Verwendung nach einem der Ansprüche 1 bis 3, dadurch gekennzeichnet, daß die basischen Calcium/Zink-Mischseifen in den üblichen Gleitmitteln in Mengen von 10 bis 60 Gew.% gelöst oder suspendiert sind.

## Claims

**1.** The use of basic calcium/zinc mixed soaps corresponding to formula (I):

$$(CaO)_n \cdot Zn(OOCR^1)_2 \qquad (I)$$

in which

$R^1$     represents one or more alkyl, alkenyl, hydroxyalkyl, hydroxyalkenyl radicals containing 7 to 21 carbon atoms or ketoalkyl radicals containing 11 to 21 carbon atoms and

$n$     is a number of 0.1 to 2.5,

the basic calcium/zinc mixed soaps being dissolved or suspended in typical lubricants for halogen containing plastics, prepared by adding calcium oxide or calcium hydroxide to a melt containing zinc soaps corresponding to formula (III):

$$Zn(OOCR^1)_2 \qquad (III),$$

where $R^1$ is as already defined,
and typical lubricants with low melt viscosities for halogen-containing plastics, the reaction mixture being heated above its melting point in the presence of catalytic quantities of acid until the free calcium oxide or calcium hydroxide has reacted off and calcium oxide or calcium hydroxide being used in a quantity of 0.1 to 2.5 moles per mole of zinc soap (III),
as stabilizers for halogen-containing thermoplastics.

**2.** The use claimed in claim 1, characterized in that basic calcium/zinc mixed soaps corresponding to formula (I), in which n is a number of 1 to 1.5, are used.

**3.** The use claimed in claim 1 or 2, characterized in that the basic calcium/zinc soaps (I) are dissolved or suspended in one or more compounds selected from the group consisting of

a) fatty alcohols containing 8 to 22 carbon atoms,
b) esters of monofunctional alkanols containing 1 to 22 carbon atoms with fatty acids containing 8 to 34 carbon atoms,
c) full esters of dicarboxylic acids containing 3 to 6 carbon atoms with monofunctional alkanols containing 8 to 22 carbon atoms,
d) partial and full esters of polyols containing 2 to 15 carbon atoms and 2 to 6 hydroxyl groups with fatty acids containing 8 to 34 carbon atoms,
e) fatty ketones corresponding to formula (II):

$$R^2\text{-CO-}R^3 \qquad (II)$$

in which $R^2$ and $R^3$ may be the same or different and represent alkyl groups containing 5 to 21 carbon atoms,
f) diamides of fatty acids containing 8 to 22 carbon atoms with alkylenediamines containing 2 to 6 carbon atoms,
g) hydrocarbon waxes, such as polyethylene waxes and paraffins,

h) oxidized polyethylene waxes with a number average molecular weight of 3,000 to 9,000.

4. The use claimed in any of claims 1 to 3, characterized in that the basic calcium/zinc mixed soaps are dissolved or suspended in the typical lubricants in quantities of 10 to 60% by weight.

**Revendications**

1. Utilisation de savon mixtes calcium /zinc basiques comme stabilisateur pour les produits thermoplastiques halogénés, dont la formule est (I)

$$(CaO)n.Zn\ (OOCR^1)_2 \hspace{3cm} (I)$$

dans laquelle $R^1$ est composé d'un ou plusieurs radicaux d'alkyle, alkényle, d'hydroxyalkyle, hydroxyalkenyle, comprenant de 7 à 21 atomes de carbones ou composés de radicaux cétoalkyle comprenant de 11 à 21 atomes de carbones, n étant compris entre 0,1 et 2,5,

- les savons mixtes calcium-zinc basiques étant dissous ou en suspension dans l'agent lubrificateur usuel pour des produits synthétiques halogénés et obtenus en ajoutant de l'oxyde de calcium et d'hydroxyde de calcium dans la masse fondue qui contient des savons de Zn de formule suivante (III)

$$Zn\ (OOCR^1)_2 \hspace{3cm} (III)$$

dont $R^1$ à la signification donnée ci--dessus et on chauffe l'agent de lubrification de faible viscosité, présent dans la masse fondue (agent lubrifiant usuel dans les produits synthétiques thermoplastiques halogénés,
- le mélange de réaction étant chauffé au delà du point de fusion, en présence de quantité catalytique d'acide jusqu'à cessation de la réaction des oxydes de calcium ou hydroxyde de calcium libres,
- on utilise une quantité d'oxyde ou d'hydroxyde de calcium comprise entre 0,1 et 2,5 mol par mol de savon Zn (III).

2. Utilisation selon la revendication I de tels savons mixtes Ca-Zn basiques de formule I, dans lesquels n est compris entre 1 et 1,5.

3. Utilisation selon la revendication 1 ou 2,
caractérisée par
le fait que ces savons mixtes ZnCa basiques sont présents dissous ou suspendus dans les groupes formés après une ou plusieurs liaisons choisies parmi :

a) alcools gras comprenant de 8 à 22 atomes de carbone
b) esters d'alcanols mono-fonctionnels comprenant de 1 à 22 atomes de carbone avec des acides gras comprenant de 8 à 34 atomes de carbone,
c) esters saturés, d'acides bicarboxyliques comprenant de 3 à 6 atomes de carbone avec des alcanols comprenant de 8 à 22 atomes de carbone,
d) esters partiels ou saturés de polyols comprenant 2 à 15 atomes de carbone ou 2 à 6 groupes d'hydroxyles, comprenant des acides gras avec 8 à 34 atomes de carbone,
e) cétones gras de formule (II)

$$R^2\text{-}CO\text{-}R^3 \hspace{3cm} (II)$$

où $R^2$ et $R^3$ sont identiques ou différents et qui représentent des groupes alcoyles comprenant 5 à 21 atomes de carbone,
f) diamides d'acides gras comprenant 8 à 22 atomes de carbone ainsi que des alcoyles diamines comprenant 2 à 6 atomes de carbone
g) cires d'hydrogène de carbone tels que cire de polyéthylène ou paraffine

h) groupes formés de cires polyéthyléniques oxydées, dont la valeur moyenne des masses molaires se situe entre 3000 et 9000.

4. Utilisation selon les revendications 1 à 3,
caractérisée en ce que
les savons mixtes, calcium-zinc, basiques représentant entre 10 et 60 % un poids sont dissous ou en suspension dans l'agent de lubrification usuel.